# EUROPÄISCHE PATENTSCHRIFT

(11) **EP 1 029 554 B1**
(45) Veröffentlichungstag und Bekanntmachung des Hinweises auf die Patenterteilung: **05.04.2006**
(21) Anmeldenummer: 99122897.4
(22) Anmeldetag: 18.11.1999
(51) Int. Cl.: A61M 1/16

(54) **Vorrichtung zur Dialysebehandlung**
Apparatus for dialysis treatment
Dispositif pour traitement de dialyse

(30) Priorität: 19.02.1999 DE 29902953 U
(43) Veröffentlichungstag der Anmeldung: 23.08.2000
(73) Patentinhaber: Fresenius Medical Care Deutschland GmbH, 61352 Bad Homburg v.d.H. (DE)
(72) Erfinder: Christmann-Braun, Horst, 61440 Oberursel (DE); Krämer, Matthias, Dr., 61381 Friedrichsdorf (DE); Van de Ven, Andreas, Dr., 35789 Weilmünster (DE)
(74) Vertreter: Luderschmidt, Schüler & Partner GbR

(56) Entgegenhaltungen:
- WO-A-98/55166
- DE-C- 19 734 992
- US-A- 4 662 208
- US-A- 5 247 434
- US-A- 5 399 157

## Beschreibung

Die Erfindung betrifft eine Vorrichtung zur Dialysebehandlung mit einem extrakorporalen Blutkreislauf, in den die Blutkammer eines durch eine semipermeable Membran in die Blutkammer und eine Dialysierflüssigkeitskammer unterteilten Dialysators geschaltet ist.

Dialysevorrichtungen der o.g. Art verfügen im allgemeinen über eine Bypassleitung , die von der mit dem Einlaß der Dialysierflüssigkeitskammer verbundenen Zuführleitung abzweigt und zu der mit dem Dialysatorauslaß verbundenen Abführleitung führt. In die Zuführ- und Abführleitung sowie die Bypassleitung sind Absperrventile geschaltet, die von einer Steuereinheit angesteuert werden. Die Steuereinheit sieht einen Betriebsmodus vor, in dem die Dialysierflüssigkeitskammer des Dialysators von dem Dialysierflüssigkeitsweg abgetrennt ist. In diesem Modus sind die Absperrventile in der Zuführ- und Abführleitung geschlossen, während das Absperrventil in der Bypassleitung geöffnet ist.

Des weiteren verfügen die bekannten Dialysevorrichtungen über eine volumetrische Bilanziereinrichtung. Die Bilanziereinrichtung weist zwei durch jeweils ein verschiebbares Element unterteilte Kammern auf, die jeweils in die Zuführ- bzw. Abführleitung geschaltet sind. Zur Bilanzierung von frischer gegen verbrauchte Dialysierflüssigkeit werden die einzelnen Bilanzkammerhälften wechselweise mit frischer und verbrauchter Dialysierflüssigkeit befüllt. Eine Dialysevorrichtung mit einer derartigen Bilanziereinrichtung ist beispielsweise aus der DE-A-28 38 414 bekannt.

Um die Dialysebehandlung optimieren zu können, ist die Ermittlung von bestimmten Parametern während der extrakorporalen Blutbehandlung notwendig. Zur Bestimmung von Parametern der Hämodialyse sind verschiedene Verfahren bekannt, die auf Konzentrationsmessungen einer Substanz sowohl im Blutkreislauf als auch im Dialysierflüssigkeitsweg beruhen. Es hat sich gezeigt, dass die Konzentration einer Substanz im Dialysierflüssigkeitsweg im wesentlichen die Konzentration der betrachteten Substanz im Blutkreislauf weitgehend unabhängig von den Behandlungsbedingungen annimmt, wenn die Flußrate der durch die Dialysierflüssigkeitskammer strömenden Dialysierflüssigkeit auf einen bestimmten Wert reduziert wird. Dies ist insofern von Vorteil, als Messungen auf der Blutseite nicht erforderlich sind.

Die Verringerung des Dialysierflüssigkeitsflusses erweist sich bei Dialysevorrichtungen, die über Bilanzkammern verfügen, als problematisch. Wenn die Taktfrequenz verringert wird, mit der die Bilanzkammern umschalten, wird ein diskontinuierlicher Flüssigkeitsfluss durch die Dialysierflüssigkeitskammer erzeugt. Bei dieser Vorgehensweise unterliegt die Temperatur der Dialysierflüssigkeit wegen der Abkühlung in den Stillstandsphasen ungewollten Schwankungen und auch die Verteilung der Elektrolyte in der Dialysierflüssigkeit ist nicht homogen, was zu starken Schwankungen in der Leitfähigkeit führt. Abgesehen von der Behandlung selbst ist dies für die Genauigkeit der Sensorik im Dialysierflüssigkeitskreislauf nachteilig.

Die DE-A-28 38 414 beschreibt eine Dialysevorrichtung, bei der die von der Bilanziervorrichtung kommende frische Dialysierflüssigkeit über einen Leitfähigkeitsmesser zu dem Dialysator strömt. Wenn die Leitfähigkeit außerhalb des zulässigen Normbereiches liegt, wird ein Bypass-Ventil umgeschaltet, so daß die Dialysierflüssigkeit nicht mehr zum Dialysator strömt, sondern auf die Abflussseite des Dialysators umgeleitet wird.

Aus der US-A-4 662 208 ist eine Vorrichtung zur Entnahme einer Dialysierflüssigkeitsprobe für eine Dialysevorrichtung bekannt. Die Entnahmevorrichtung verfügt über jeweils eine von der Dialysierflüssigkeitszuführ- und abführleitung abzweigende Probenentnahmeleitung, in der jeweils eine Pumpe angeordnet ist, deren Flussrate veränderbar ist. Zur Entnahme einer Probe stromauf oder stromab des Dialysators wird entweder die eine oder andere Pumpe betrieben.

Der Erfindung liegt die Aufgabe zugrunde, eine Vorrichtung zur Dialysebehandlung zu schaffen, bei der sich der Dialysierflüssigkeitsfluss ohne großen apparativen Aufwand auf einfache Weise verringern lässt.

Die Lösung dieser Aufgabe erfolgt mit den Merkmalen des Patentanspruchs 1.

Zur Verringerung des Dialysierflüssigkeitsflusses kann bei der erfindungsgemäßen Vorrichtung von den Mitteln Gebrauch gemacht werden, die in den bekannten Dialysevorrichtungen ohnehin in der Bypassleitung und der Zuführleitung und/oder Abführleitung vorgesehen sind, wodurch der apparative Aufwand gering gehalten wird.

In dem Betriebsmodus für den verringerten Dialysierflüssigkeitsfluß werden die Mittel zum Einstellen des Dialysierflüssigkeitsflusses derart angesteuert, daß die Dialysierflüssigkeit sowohl durch die Bypassleitung als auch die mit der Dialysierflüssigkeitskammer verbundene Zuführ- und Abführleitung strömt. Der Fluß durch die Dialysierflüssigkeitskammer wird also dadurch verringert, daß ein Teil der Dialysierflüssigkeit über die Bypassleitung umgeleitet wird. Da die Dialyierflüssigkeitskammer kontinuierlich von Dialysierflüssigkeit durchströmt wird, ergeben sich keine Probleme bei der Temperaturregelung. Auch die Zusammensetzung und Homogenität der Dialysierflüssigkeit entspricht genau den Verhältnissen während der Dialysebehandlung. Bei konstantem Hauptfluß ist eine Umschaltung auf den langsamen Fluß ohne Verzögerung durch Einschwingvorgänge möglich. Nach der Bestimmung des hämodynamischen Parameters bei verringertem Dialysierflüssigkeitsfluß kann die Behandlung mit normalem Fluß sofort fortgesetzt werden, ohne daß sich erst eine konstante Leitfähigkeit wieder einstellen muß.

Die von der Zuführleitung stromauf der Dialysierflüssigkeitskammer abzweigende Bypassleitung kann zu der Abführleitung stromab des Dialysators führen. Es ist aber auch möglich, daß die Bypassleitung in einen Abfluß mündet. Die Flüsse in den Leitungen können mittels Durchflußsensoren und/oder Pumpen erfaßt werden.

Eine Veränderung der Leitungsführung der bekannten Dialysevorrichtungen ist dann nicht erforderlich, wenn die Mittel zum Einstellen des Dialysierflüssigkeitsflusses das in die Bypassleitung geschaltete Bypassventil und das in die Zuführ- und/oder Abführleitung geschaltete Dialysatorventil der Dialysevorrichtung umfassen. Wenn sowohl das Bypassventil als auch das in die Zuführ- bzw. Abführleitung geschaltete Dialysatorventil geöffnet werden, erfolgt eine Aufteilung des Hauptstromes, der zu einer Verringerung des Dialysierflüssigkeitsflusses durch die Dialysierflüssigkeitskammer führt, wobei die Größe des Dialysierflüssigkeitsstroms von den Strömungsverhältnissen in der Dialysevorrichtung abhängig ist.

Die Mittel zum Einstellen des Dialysierflüssigkeitsflusses können nicht nur als Ventile, sondern auch als Pumpen ausgebildet sein. Wenn die Pumpen stillstehen, wird keine Flüssigkeit gefördert, während bei laufenden Pumpen Flüssigkeit durch die Leitungen fließt.

Bei einer bevorzugten Ausführungsform ist eine zweite Bypassleitung vorgesehen, die das Bypassventil in der ersten Bypassleitung überbrückt, wobei in die zweite Bypassleitung ein zweites Bypassventil und eine Drossel geschaltet sind. Zur Verringerung des Dialysierflüssigkeitsflusses werden das erste Bypassventil geschlossen und das zweite Bypassventil und das in die Zuführ- bzw. Abführleitung geschaltete Dialysatorventil geöffnet. Diese Ausführungsform hat den Vorteil, daß der Fluß durch die Dialysierflüssigkeitskammer mit der Drossel vergrößert werden kann, falls sich ansonsten aufgrund der vorgegebenen Strömungsverhältnisse in der Dialysevorrichtung ein zu geringer Dialysierflüssigkeitsfluß im Dialysator einstellen sollte.

In der Praxis hat sich jedoch gezeigt, daß aufgrund des Strömungswiderstandes der Dialysatorventile allein durch das Öffnen des ersten Bypassventils bei geöffneten Dialysatorventilen sich ein Dialysierflüssigkeitsfluß durch die Dialysierflüssigkeitskammer einstellt, der für eine ausreichende Angleichung der Dialysierflüssigekeitskonzentration auf die Blutwerte der betrachteten Substanzen eher zu groß ist.

Um den Dialysierflüssigkeitsfluß auf geringere Werte einstellen zu können, ist bei einer weiteren bevorzugten Ausführungsform eine zweite Bypassleitung vorgesehen, die das in die Zuführ- oder Abführleitung geschaltete Dialysatorventil überbrückt, wobei in die zweite Bypassleitung ein zweites Bypassventil und eine Drossel geschaltet sind. Bei dieser Ausführungsform werden das erste und zweite Bypassventil geöffnet und das in die Zuführ- bzw. Abführleitung geschaltete Dialysatorventil geschlossen. Der Dialysierflüssigkeitsfluß durch die Dialysierflüssigkeitskammer wird nun durch den Strömungswiderstand der Drossel in dem zweiten Bypass-Zweig bestimmt.

Die Ausführungsform, bei der das stromauf der Dialysierflüssigkeitskammer angeordnete Dialysatorventil überbrückt wird, ist insofern besonders vorteilhaft, als der Dialysator auf der Seite der Drossel mit dem niedrigeren Druck liegt, wodurch die Gefahr der "Backfiltration" geringer ist.

Die Drossel in der zweiten Bypassleitung kann sowohl eine Drossel mit festem als auch eine Drossel mit einstellbarem Strömungswiderstand sein.

Bei einer weiteren bevorzugten Ausführungsform ist der Strömungswiderstand der Drossel einstellbar, wobei der Dialysierflüssigkeitsfluß durch die Dialysierflüssigkeitskammer gemessen wird. Der Strömungswiderstand der Drossel wird so geregelt, daß sich bei einem vorgegebenen Hauptfluß ein bestimmter Dialysierflüssigkeitsfluß durch die Dialysierflüssigkeitskammer einstellt.

Die Entnahme einer Probe erfolgt vorteilhafterweise mit einer Probenentnahmepumpe, die bei verringertem Dialysierflüssigkeitsfluß stromab der Dialysierflüssigkeitskammer Dialysierflüssigkeit ansaugt und wieder in das bilanzierende System zurückpumpt. Zur Umleitung der Dialysierflüssigkeit ist eine erste Probenentnahmeleitung, die von der Abführleitung stromauf des in dieselbe geschalteten Dialysatorventils zu dem einen Anschluß der Probenentnahmepumpe führt, und eine zweite Probenentnahmeleitung vorgesehen, die von dem anderen Anschluß der Pumpe zu der Abführleitung stromab des in dieselbe geschalteten Dialysatorventils führt. Die zweite Probenentnahmeleitung kann auch in die Bypassleitung oder jede andere Leitung führen, die mit der Abführleitung stromab des Dialysatorventils verbunden ist. Da die Dialysierflüssigkeit in das bilanzierende System zurückfließt, wird die Bilanzierung nicht beeinträchtigt. Nach Einstellung eines Gleichgewichtszustandes in der Dialysierflüssigkeit kann über eine dritte Probenentnahmeleitung, die mit der ersten oder zweiten Probenentnahmeleitung verbunden ist, eine definierte Menge an Dialysierflüssigkeit entnommen und zu Mitteln zum Bestimmen hämodynamischer Parameter geleitet werden. Als hämodynamische Parameter kann beispielsweise eine Konzentration bestimmt werden. Die entnommene Menge an Dialysierflüssigkeit kann bei der Bilanzierung berücksichtigt werden.

Die Probenentnahmepumpe kann eine genau definierte Menge an Dialysierflüssigkeit mit geringer Geschwindigkeit durch den Dialysator fördern, unabhängig von aktuellen Dialysatflüssen im Hydraulikkreislauf und mit genügender Genauigkeit unabhängig vom jeweils herrschenden Druck, Dialysatortyp und Ausrüstungszustand der Dialysevorrichtung. Die Pumpe kann auch zur Förderung von Probenlösung der normalen Dialyse sowie zum Spülen und Reinigen der Strömungskanäle eingesetzt werden.

Die Probenentnahmepumpe sowie die damit zusammenhängenden Komponenten können in einer ansteckbaren Kassetteneinheit zusammengefaßt sein, die sich an den Hydraulikkreislauf einer bekannten Dialysevorrichtung koppeln läßt. Über Ventile können die Pumpe oder die anderen Komponenten im Falle einer Störung vom Hydraulikkreislauf getrennt werden, wobei die Dialyse unbeeinflußt weiter laufen kann.

Eine weitere bevorzugte Ausführungsform sieht zur Vereinfachung des Anschlusses der Kassetteneinheit an den Hydraulikkreislauf der bekannten Dialysevorrichtung nur zwei Anschlußleitungen vor.

Im folgenden werden mehrere Ausführungsbeispiele der Erfindung unter Bezugnahme auf die Zeichnungen näher erläutert.

Es zeigen:
- Figur 1: eine vereinfachte schematische Darstellung einer ersten Ausführungsform einer Hämodialysevorrichtung,
- Figur 2: eine zweite Ausführungsform der Dialysevorrichtung,
- Figur 3: eine dritte Ausführungsform der Dialysevorrichtung,
- Figur 4: eine vierte Ausführungsform der Dialysevorrichtung,
- Figur 5: ein erstes Ausführungsbeispiel einer Analyseeinheit zusammen mit den wesentlichen Komponenten einer Dialysevorrichtung in vereinfachter schematischer Darstellung,
- Figur 6: eine vereinfachte schematische Darstellung eines zweiten Ausführungsbeispiels einer Analyseeinheit zusammen mit den wesentlichen Komponenten der Dialysevorrichtung und
- Figur 7: eine vereinfachte schematische Darstellung eines dritten Ausführungsbeispiels einer Analyseeinheit.

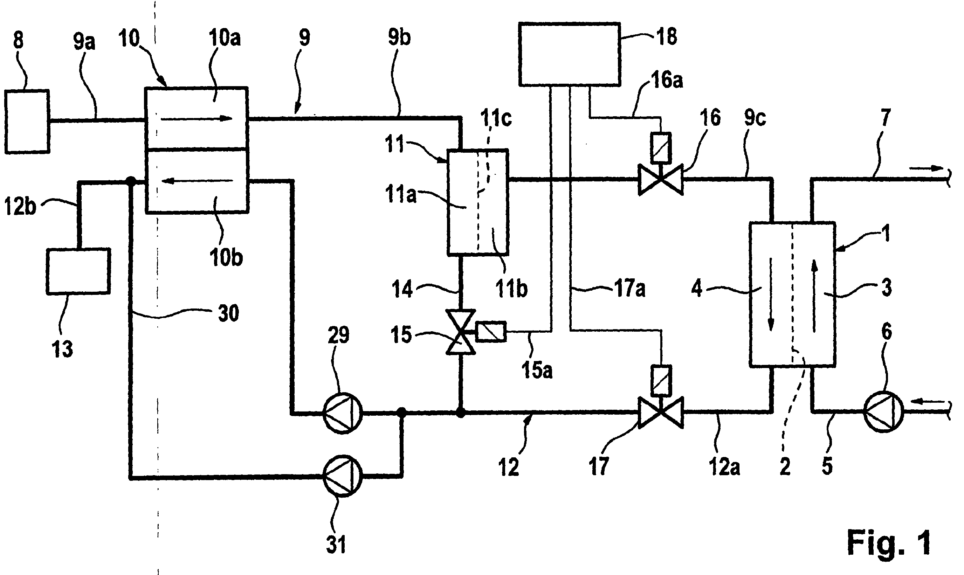

In Figur 1 sind nur die wesentlichen Komponenten der Hämodialysevorrichtung dargestellt. Die Dialysevorrichtung weist einen Dialysator 1 auf, der durch eine semipermeable Membran 2 in eine Blutkammer 3 und eine Dialysierflüssigkeitskammer 4 unterteilt ist. An dem Einlaß der Blutkammer 3 ist eine arterielle Blutleitung 5 angeschlossen, in die eine Blutpumpe 6 geschaltet ist. Von dem Auslaß der Blutkammer führt eine venöse Blutleitung 7 zu dem Patienten.

In einer Dialysierflüssigkeitsquelle 8 wird frische Dialysierflüssigkeit bereitgestellt. Von der Dialysierflüssigkeitsquelle 8 führt ein erster Leitungsabschnitt 9a einer Dialysierflüssigkeitszuführleitung 9 zu einen Einlaß einer in zwei Bilanzkammerhälften 10a, b unterteilten Bilanziereinrichtung 10 zur Bilanzierung frischer gegen verbrauchte Dialysierflüssigkeit. In einer volumetrisch bilanzierenden Dialysemaschine werden im allgemeinen zwei parallel geschaltete Bilanzkammern verwendet, von denen in Figur 1 der Einfachheit halber aber nur eine gezeigt ist. Von dem Auslaß der ersten Bilanzkammer führt ein zweiter Leitungsabschnitt 9b der Zuführleitung 9 zu dem Einlaß der ersten Kammer 11 a eines durch eine Keime zurückhaltende Membran 11c in zwei Kammern 11a,b unterteilten Sterilfilters 11, der in den Dialysierflüssigkeitsweg geschaltet ist, um die Dialysierflüssigkeit von Keimen zu befreien. Von dem Auslaß der zweiten Kammer 11b des Sterilfilters 11 führt ein dritter Leitungsabschnitt 9c der Zuführleitung zu dem Einlaß der Dialysierflüssigkeitskammer 4. Von dem Auslaß der Dialysierflüssigkeitskammer 4 führt der erste Leitungsabschnitt 12a einer Dialysierflüssigkeitsabführleitung 12 zu dem Einlaß der zweiten Kammer 10b der Bilanziereinrichtung . Der Auslaß der zweiten Bilanzierkammer 10b ist über einen zweiten Leitungsabschnitt 12b der Abführleitung mit einem Abfluß 13 verbunden. In den ersten Leitungsabschnitt 12a der Dialysierflüssigkeitsabführleitung 12 ist eine Dialysierflüssigkeitspumpe 29 geschaltet. Stromauf der Dialysier-flüssigkeitspumpe 29 zweigt von dem ersten Leitungsabschnitt 12a eine Ultrafiltrationsleitung 30 ab, die zu dem zweiten Leitungsabschnitt 12b der Dialysierflüssigkeitsabführleitung 12 führt. In die Ultrafiltrationsleitung 30 ist eine Ultrafiltrationspumpe 31 geschaltet.

Zur Abtrennung der Dialysierflüssigkeitskammer 4 des Dialysators von dem Dialysierflüssigkeitsweg verfügt die Dialysevorrichtung über eine Bypassleitung 14, die von dem Auslaß der ersten Kammer 11a des Sterilfilters 11 zu dem ersten Leitungsabschnitt 12a der Abführleitung führt. In die Bypassleitung 14 ist ein elektromagnetisch betätigbares Bypassventil 15 geschaltet, während in den dritten Leitungsabschnitt 9c der Zuführleitung ein erstes Dialysatorventil 16 und in den ersten Leitungsabschnitt 12a der Abführleitung ein zweites Dialysatorventil 17 geschaltet ist. Die beiden Dialysatorventile 16, 17 sind ebenfalls elektromagnetisch betätigbar. Das Bypassventil 15 und die beiden Dialysatorventile 16, 17 werden von einer zentralen Steuereinheit 18 angesteuert, die über Steuerleitungen 15a, 16a, 17a mit den Ventilen verbunden ist. Die zentrale Steuereinheit 18 gibt verschiedene Betriebsmodi vor. Zur Abkopplung des Dialysators 1 von dem Dialysierflüssigkeitsweg steuert die Steuereinheit die Ventile derart an, daß das Bypassventil 15 bei geschlossenen Dialysatorventilen 16, 17 geöffnet ist. Darüber hinaus sieht die Steuereinheit einen Betriebsmodus für einen verringerten Dialysierflüssigkeitsfluß vor, um Messungen im Dialysierflüssigkeitsweg zum Bestimmen hämodynamischer Parameter durchführen zu können. In diesem Betriebsmodus steuert die Steuereinheit die Ventile derart an, daß das Bypassventil 15 und die Dialysatorventile 16, 17 geöffnet sind, so daß die Dialysierflüssigkeit sowohl durch die Bypassleitung 14 als auch durch die Dialysierflüssigkeitskammer 4 strömt.

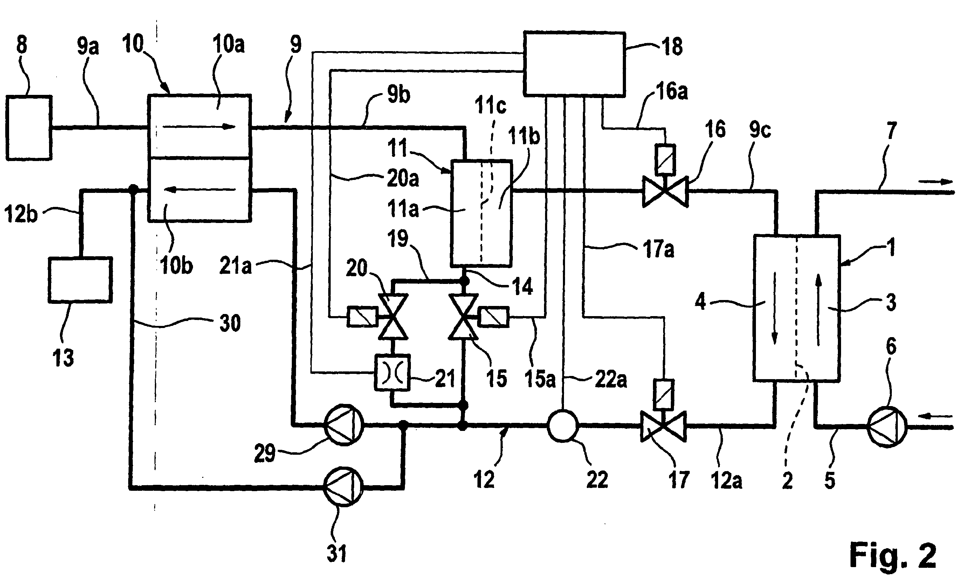

Die Ausführungsform von Figur 2 unterscheidet sich von dem unter Bezugnahme auf Figur 1 beschriebenen Ausführungsbeispiel durch eine weitere Bypassleitung mit einem zweiten Bypassventil und einer Drossel. Die Teile der Dialysevorrichtung von Figur 2, die denjenigen der Vorrichtung von Figur 1 entsprechen, sind mit den gleichen Bezugszeichen versehen. Die zweite Bypassleitung 19 zweigt von dem Leitungsabschnitt der ersten Bypassleitung 14 stromauf des ersten Bypassventils 15 ab und führt zu dem Leitungsabschnitt der ersten Bypassleitung stromab des Bypassventils. Die zweite Bypassleitung 19 enthält ein elektromagnetisch betätigbares zweites Bypassventil 20 und eine Drossel 21, deren Strömungsquerschnitt einstellbar ist. Das zweite Bypassventil 20 und die Drossel werden von der Steuereinheit 18 über Steuerleitungen 20a, 21a angesteuert. Darüber hinaus verfügt die Dialysevorrichtung über einen Flußmesser 22, der stromab des zweiten Dialysatorventils 17 in den ersten Leitungsabschnitt 12a der Abführleitung geschaltet ist. Prinzipiell ist es aber auch möglich, daß der Flußmesser zur Überwachung des Dialysierflüssigkeitsflusses durch die Dialysierflüssigkeitskammer des Dialysators in den dritten Leitungsabschnitt 9c der Zuführleitung 9 geschaltet ist. Auch kann der Flußmesser in der ersten Bypassleitung 14 angeordnet sein, in diesem Fall ist der Dialysierflüssigkeitsfluß allerdings durch Bildung der Differenz von Hauptfluß und dem Fluß durch die erste Bypassleitung zu berechnen. Der Flußmesser 22 ist mit einer Datenleitung 22a mit der Steuereinheit 18 verbunden.

In dem Betriebsmodus für den verringerten Dialysierflüssigkeitsfluß schließt die Steuereinheit 18 das erste Bypassventil 15, während das zweite Bypassventil 20 und die beiden Dialysatorventile 16, 17 geöffnet werden. Die Dialysierflüssigkeit strömt nun nicht mehr durch die erste, sondern die zweite Bypassleitung, wobei die Strömungsrate durch den Strömungsquerschnitt der Drossel 21 bestimmt wird. Die Steuereinheit 18 stellt den Strömungsquerschnitt der Drossel derart ein, daß der Dialysierflüssigkeitsfluß durch die Dialysierflüssigkeitskammer 4 einen vorgegebenen Wert annimmt, der von dem Flußmesser 22 überwacht wird.

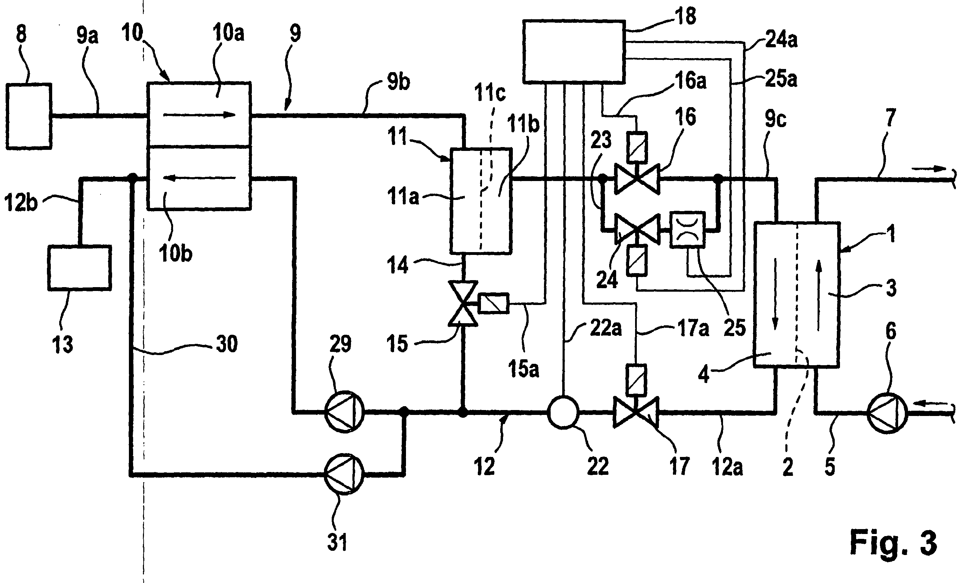

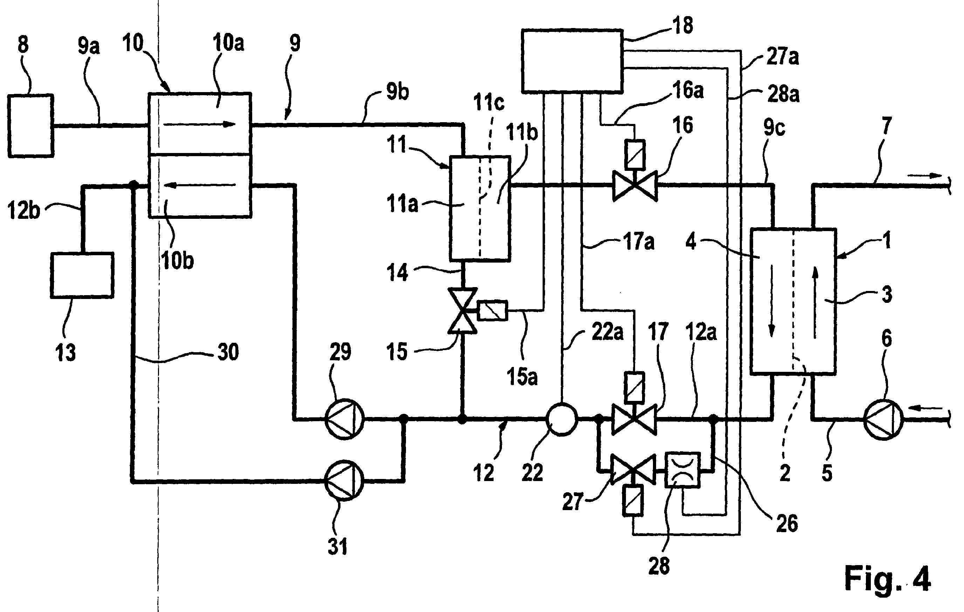

Die Figuren 3 und 4 zeigen zwei weitere Ausführungsbeispiele, bei denen ein zweiter Bypass-Zweig nicht zur Überbrückung des ersten Bypassventils 15, sondern des ersten oder zweiten Dialysatorventils vorgesehen ist. Die einander entsprechenden Teile der Dialysevorrichtung sind wieder mit den gleichen Bezugszeichen versehen.

Bei der Ausführungsform von Figur 3 zweigt die zweite Bypassleitung 23 von dem dritten Leitungsabschnitt 9c der Zuführleitung 9 stromauf des ersten Dialysatorventils 16 ab und führt zu dem dritten Zuführleitungsabschnitt 9c stromab des Dialysatorventils 16. Das zweite Bypassventil und die Drossel sind mit den Bezugszeichen 24, 25 versehen. Sie werden über die Steuerleitungen 24a, 25a angesteuert.

Bei dem Ausführungsbeispiel von Figur 4 zweigt die zweite Bypassleitung 26 von dem ersten Leitungsabschnitt 12a der Abführleitung stromauf des zweiten Dialysatorventils 17 ab und führt zu dem Abführleitungsabschnitt 12a stromab des Dialysatorventils 17. Das zweite Bypassventil und die Drossel sind mit den Bezugszeichen 27 bzw. 28 und die Steuerleitungen mit den Bezugszeichen 27a bzw. 28a versehen.

In dem Betriebsmodus für den verringerten Dialysierflüssigkeitsfluß öffnet die Steuereinheit 18 das erste Bypassventil 15 und schließt eine der beiden Dialysatorventile 16, 17, während das zweite Bypassventil 24 bzw. 27 geöffnet wird. Der Dialysierflüssigkeitsfluß wird nun durch den Strömungsquerschnitt der Drossel 25 bzw. 28 vorgegeben. Die Steuereinheit stellt den Strömungsquerschnitt so ein, daß der Dialysierflüssigkeitsfluß den vorgegebenen Wert annimmt, der wieder mit dem Flußmesser 22 überwacht wird.

Prinzipiell ist es auch möglich, daß nur ein Dialysatorventil entweder stromauf oder stromab der Dialysierflüssigkeitskammer 4 vorgesehen ist. Auch können anstelle der Drossel mit veränderbarem Strömungsquerschnitt Drosseln mit konstantem Strömungsquerschnitt vorgesehen sein. In diesem Fall kann allerdings eine Flußregelung nicht erfolgen, was aber für die erforderlichen Messungen vielfach ausreichend ist.

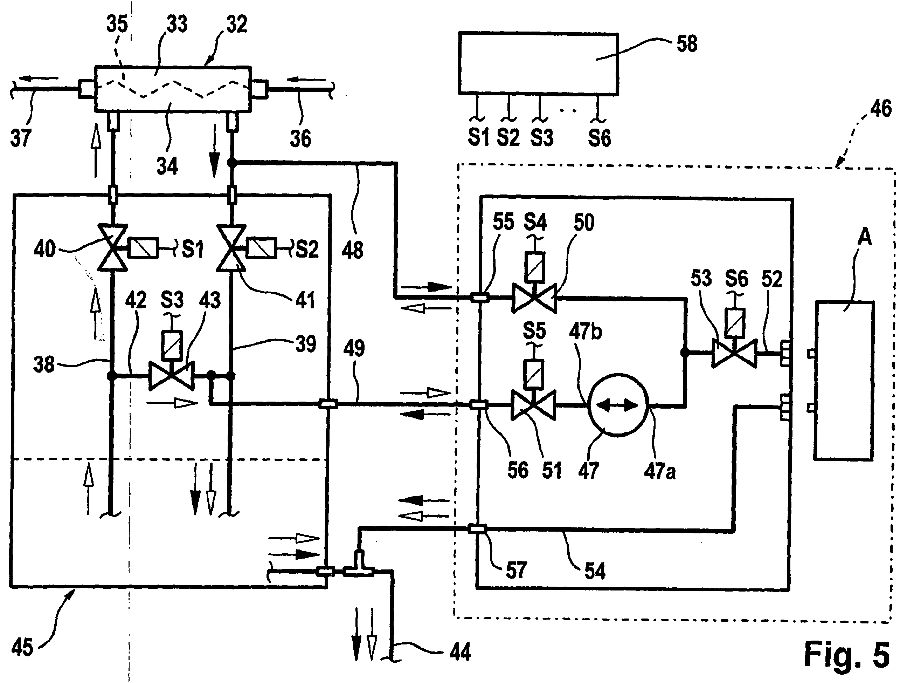

Figur 5 zeigt eine als austauschbare Kassetteneinheit ausgebildete Analyseeinheit zusammen mit den wesentlichen Komponenten einer Dialysevorrichtung. Die Dialysevorrichtung verfügt wieder über einen Dialysator 32 mit einer Blutkammer 33 und einer Dialysierflüssigkeitskammer 34, die von einer semipermeablen Membran 35 voneinander getrennt sind. Über die Blutzuführleitung 36 strömt Blut in die Blutkammer 33 und über die Blutat führleitung 37 aus der Blutkammer 33. Die Dialysierflüssigkeit strömt aus einer nicht dargestellten Bilanziereinrichtung über die Dialysierflüssigkeits-Zuführleitung 38 in die Dialysierflüssigkeitskammer 34 und über eine Dialysierflüssigkeits-Abführleitung 39 aus der Dialysierflüssigkeitskammer 34 zurück zu der Bilanziereinrichtung. In die Zuführleitung 38 ist ein erstes Dialysatorventil 40 und in die Abführleitung ein zweites Dialysatorventil 41 geschaltet. Eine Bypassleitung 42, in die ein Bypassventil 43 geschaltet ist, verbindet die Zuführleitung 38 mit der Abführleitung 39. Die verbrauchte Dialysierflüssigkeit wird über eine Abflußleitung 44 abgeführt. Die Zuführ-, Abführ- und Bypassleitung mit den Ventilen sowie die Bilanziereinrichtung bilden den Hydraulikteil 45 der Dialysevorrichtung.

Die austauschbare Kassetteneinheit 46 umfaßt eine Probenentnahmepumpe 47 sowie Mittel zum Bestimmen hämodynamischer Parameter, beispielsweise eine Analyseeinrichtung A mit Sensoren zur Bestimmung der Konzentration von harnpflichtigen Substanzen, z. B. Harnstoff, in der Dialysierflüssigkeit. Von der Abführleitung 39 zweigt stromauf des Dialysatorventils 41 eine erste Probenentnahmeleitung 48 ab, die zu dem einen Anschluß 47a der Probenentnahmepumpe 47 führt, bei der es sich um eine bidirektionale Membranpumpe handelt. Von dem anderen Anschluß 47b der Pumpe 47 führt eine zweite Probenentnahmeleitung 49 zu der Bypassleitung 42 stromab des Bypassventils 43. In die erste und zweite Entnahmeleitung 48, 49 sind jeweils ein Probenentnahmeventil 50, 51 geschaltet. Eine dritte Probenentnahmeleitung 52, in die ein weiteres Probenentnahmeventil 53 geschaltet ist, zweigt von der ersten Entnahmeleitung 48 ab und führt zu dem Einlaß der Analyseeinrichtung A. Von dem Auslaß der Analyseeinrichtung A führt eine Auslaßleitung 54 zu der Abflußleitung 44. Die Probenentnahmeleitungen sowie die Auslaßleitung bestehen jeweils aus zwei Leitungsabschnitten, die über Konnektoren 55, 56, 57 miteinander verbunden werden können, um die Kassetteneinheit 46 von dem Hydraulikteil 45 der Dialysevorrichtung abnehmen zu können. Zur Ansteuerung der elektromagnetisch betätigbaren Ventile ist eine Steuereinheit 58 vorgesehen, die über Steuerleitungen S 1 bis S6 mit den Ventilen verbunden ist.

Während des Dialysebetriebs öffnet die Steuereinheit 58 das erste und zweite Dialysatorventil 40, 41 und schließt das Bypassventil 43 sowie die Probenentnahmeventile 50, 51, 53. Zur Entnahme einer Probe werden das Bypassventil 43 geöffnet und das zweite Dialysatorventil 41 geschlossen und das erste und zweite Probenentnahmeventil 50, 51 abwechselnd geöffnet. Die Pumpe 47 saugt bei geöffnetem ersten und geschlossenem zweiten Probenentnahmeventil eine definierte Menge abfließende Dialysierflüssigkeit an und pumpt bei geöffnetem zweiten und geschlossenem ersten Ventil die verbrauchte Dialysierflüssigkeit zurück in das bilanzierende System. Nachdem sich ein Gleichgewichtzustand eingestellt hat, wird bei geschlossenem ersten und zweiten Entnahmeventil 50, 51 das dritte Ventil 53 geöffnet, so daß eine Dialysierflüssigkeitsprobe in die Analyseeinrichtung A fließt. Aus der Analyseeinrichtung A wird die Probe dann in den Abfluß geleitet. Wenn die frische Dialysierflüssigkeit über die Bypassleitung 42 fließt, kann ein niedriger Dialysierflüssigkeitsfluß im Dialysator eingestellt werden, ohne die Hydrauliksteuerung der Dialysevorrichtung negativ zu beeinflussen. Leitfähigkeit und Temperatur bleiben somit konstant. Da die Pumpe bidirektional arbeitet, können nach einer Flußumkehrung durch entsprechende Schaltung der Probenentnahmeventile auch Proben von der frischen Dialysierflüssigkeit entnommen werden. Die definierte Menge an entnommener Probenflüssigkeit kann bei der Bilanzierung berücksichtigt werden.

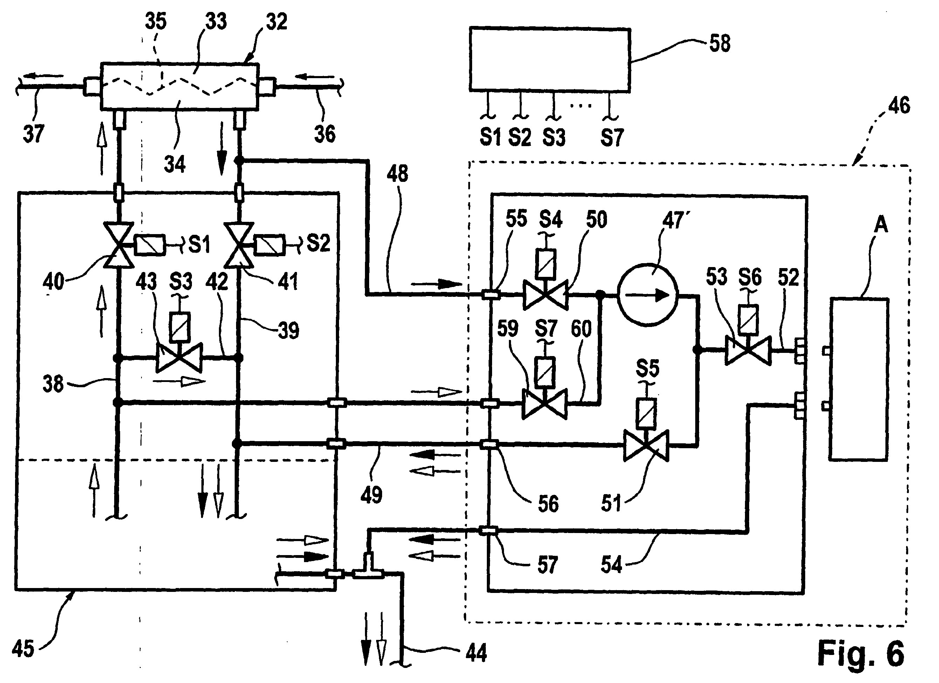

Figur 6 zeigt ein weiteres Ausführungsbeispiel, das sich von der unter Bezugnahme auf Figur 5 beschriebenen Ausführungsform dadurch unterscheidet, daß die Probenentnahmepumpe nur unidirektional arbeitet. Bei diesem Ausführungsbeispiel ist die Entnahme einer Probe von der frischen Dialysierflüssgkeit auch ohne Umkehrung der Flußrichtung möglich. Die einander entsprechenden Teile der Ausführungsformen gemäß der Figuren 5 und 6 sind mit den gleichen Bezugszeichen versehen. Von der Zuführleitung 38 zweigt bei dem Ausführungsbeispiel gemäß Figur 6 eine vierte Probenentnahmeleitung 60 ab, die zu der ersten Entnahmeleitung 48 stromauf der Entnahmepumpe 47' führt. In die vierte Probenentnahmeleitung 60 ist ein viertes Probenentnahmeventil 59 geschaltet. Ein weiterer Unterschied ist, daß die dritte Probenentnahmeleitung 52 nicht von der ersten Entnahmeleitung 48, sondern von der zweiten Entnahmeleitung 49 abzweigt. Das vierte Probenentnahmeventil 59 ist über eine weitere Steuerleitung S7 mit der Steuereinheit 58 verbunden und wird von der Steuereinheit 58 während des Dialysebetriebs sowie der Entnahme einer Probe von der abfließenden Dialysierflüssigkeit geschlossen. Die Ansteuerung der übrigen Ventile erfolgt, wie bei dem Ausführungsbeispiel gemäß Figur 5. Für die Entnahme einer Probe_von der frischen Dialysierflüssigkeit allerdings, wird das erste Probenentnahmeventil 50 geschlossen und das vierte Entnahmeventil 59 geöfnet, so daß frische Dialysierflüssigkeit von der Entnahmepume 47 angesaugt wird.

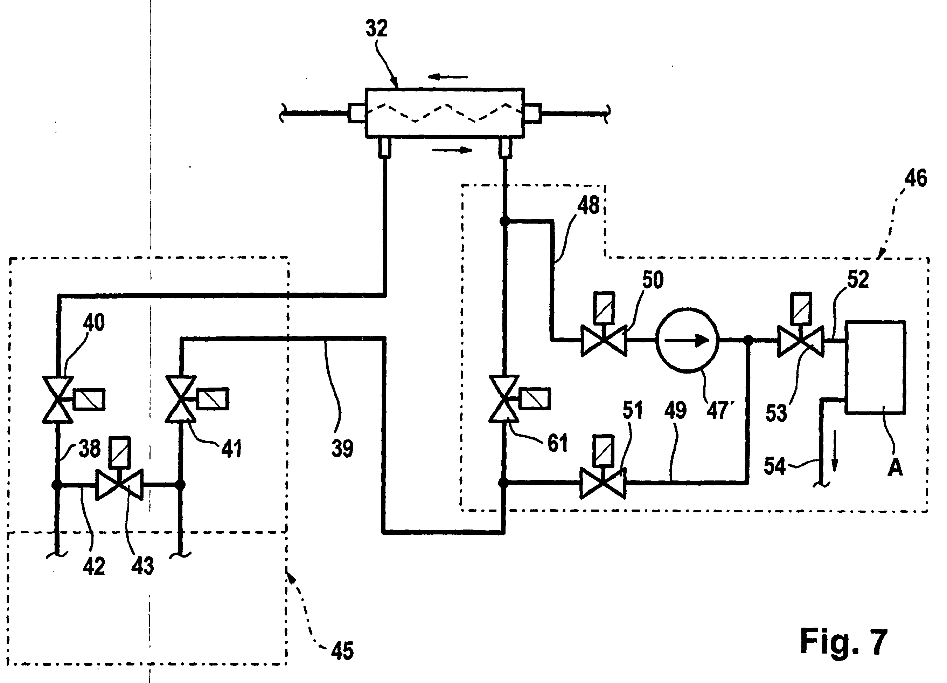

Eine weitere Ausführungsform zeigt Figur 7, die eine Anbindung der austauschbaren Kassetteneinheit an den Hydraulikteil der Dialysevorrichtung mit nur zwei Anschlußleitungen ermöglicht. Die Teile des Ausführungsbeispiels gemäß Figur 7, die denen der Ausführungsformen gemäß der Figuren 5 und 6 entsprechen, sind wieder mit den gleichen Bezugszeichen versehen. Der Hydraulikteil 45 umfaßt die zu dem Dialysator 32 führende Zuführleitung 38 mit dem ersten Dialysatorventil 40 und die von dem Dialysator abgehende Abführleitung 39 mit dem zweiten Dialysatorventil 41 sowie die Bypassleitung 42 mit dem Bypassventil 43. Zwischen dem erstem Dialysatorventil 41 und dem Dialysator 32 ist in die Abführleitung 39 ein drittes Dialysatorventil 61 geschaltet.

Während des Dialysebetriebs sind das erste, zweite und dritte Dialysatorventil geöffnet, während das Bypassventil 43 geschlossen ist.

Die erste Probenentnahmeleitung 48 zweigt von der Abführleitung 39 stromauf des dritten Dialysatorventils 61 ab und führt zu der unidirektionalen Entnahmepumpe 47', bei der es sich ebenfalls um eine Membranpumpe handelt. Von der Entnahmepumpe 47' führt die zweite Probenentnahmeleitung 49 zu der Abführleitung 39 stromab des dritten Dialysatorventils 61. Die dritte Entnahmeleitung 52, die von der zweiten Entnahmeleitung 49 abzweigt, führt zu der Analyseeinrichtung A, von der die Auslaßleitung 54 abgeht. In die erste, zweite und dritte Probenentnahmeleitung sind das erste, zweite und dritte 50, 51, 53 Probenentnahmeventil geschaltet. Die Steuereinheit sowie die Steuerleitungen sind in Figur 7 nicht gezeigt.

Zur Probenentnahme wird das dritte Dialysatorventil 61 geschlossen, so daß die Dilaysierflüssigkeit über die erste und zweite Probenentnahmeleitung 48, 49 fließt. Das erste und zweite Probenentnahmeventil 50, 51 werden abwechselnd geöffnet bzw. geschlossen, wobei die Probenentnahmepumpe 47' eine definierte Menge an Dialysierflüssigkeit fördert, die bei geschlossenen Entnahmeventilen 50, 51 und geöffnetem Ventil 53 der Analyseeinrichtung A nach Einstellung des Gleichgewichtszustandes zugeführt werden kann.

## Patentansprüche

1. Vorrichtung zur Dialysebehandlung mit
einem extrakorporalen Blutkreislauf, in den die Blutkammer (3) eines durch eine semipermeable Membran (2) in die Blutkammer und eine Dialysierflüssigkeitskammer (4) unterteilten Dialysators (1) geschaltet ist,
einer Dialysierflüssigkeit-Zuführleitung (9) eines Dialysierflüssigkeitsweges, die mit dem Einlass der Dialysierflüssigkeitskammer verbunden ist und einer Dialysierflüssigkeit-Abführleitung (12), die mit dem Auslass der Dialysierflüssigkeitskammer verbunden ist,
einer Bilanziereinrichtung (10),
einer Bypassleitung (14), die stromauf der Dialysierflüssigkeitskammer von der Zuführleitung abzweigt,
Mitteln zum Einstellen des Dialysierflüssigkeitsflusses (15,16,17;20,21;24,25;27,28), die sowohl in der Bypassleitung als auch in der Zuführ- und/oder Abführleitung vorgesehen sind, und
Mitteln (18) zum Ansteuern der Mittel zum Einstellen des Dialysierflüssigkeitsflusses in der Bypassleitung und der Zuführleitung und/oder Abführleitung,
**dadurch gekennzeichnet,**
**dass** die Mittel (18) zum Ansteuern der Mittel zum Einstellen des Dialysierflüssigkeitsflusses (15,16,17;20,21;24,25;27,28) derart ausgebildet sind, dass ein Betriebsmodus für einen verringerten Dialysierflüssigkeitsfluss durch die Dialysierflüssigkeitskammer (4) einstellbar ist, bei dem die Mittel zum Einstellen des Dialysierflüssigkeitsflusses sowohl in der Bypassleitung (14) als auch der Zuführ- und Abführleitung (9, 12) in eine den Dialysierflüssigkeitsfluss sowohl durch die Bypassleitung als auch durch die Zuführ- und Abführleitung zumindest teilweise freigebende Stellung angesteuert sind, so dass ein Teil der Dialysierflüssigkeit über die Bypassleitung (14) umleitbar ist.

2. Vorrichtung nach Anspruch 1, **dadurch gekennzeichnet, dass** die Mittel zum Einstellen des Dialysierflüssigkeitsflusses ein in die Bypassleitung geschaltetes Bypassventil (15) und ein in die Zuführleitung (9) geschaltetes Dialysatorventil (16) und/oder ein in die Abführleitung (12) geschaltetes Dialysatorventil (17) umfassen.

3. Vorrichtung nach Anspruch 2, **dadurch gekennzeichnet, dass** die Mittel zum Ansteuern der Mittel zum Einstellen des Dialysierflüssigkeitsflusses eine Steuereinheit (18) umfassen, die derart ausgebildet ist, dass in dem Betriebsmodus für den verringerten Dialysierflüssigkeitsfluss das Bypassventil (15) und das in die Zuführleitung geschaltete bzw. in die Abführleitung geschaltete Dialysatorventil (16,17) geöffnet sind.

4. Vorrichtung nach Anspruch 2, **dadurch gekennzeichnet, dass** eine zweite Bypassleitung (19) vorgesehen ist, die von der ersten Bypassleitung (14) stromauf des ersten Bypassventils (15) abzweigt und stromab des ersten Bypassventils zu der ersten Bypassleitung führt, wobei in die zweite Bypassleitung ein zweites Bypassventil (20) und eine Drossel (21) geschaltet sind.

5. Vorrichtung nach Anspruch 4, **dadurch gekennzeichnet, dass** die Mittel zum Ansteuern der Mittel zum Einstellen des Dialysierflüssigkeitsflusses eine Steuereinheit (18) umfassen, die derart ausgebildet ist, dass in den Betriebsmodus für den verringerten Dialysierflüssigkeitsfluss das erste Bypassventil (15) geschlossen und das zweite Bypassventil (20) und das in die Zuführleitung- bzw. Abführleitung geschaltete Dialysatorventil (16,17) geöffnet sind.

6. Vorrichtung nach Anspruch 2, **dadurch gekennzeichnet, dass** eine zweite Bypassleitung (23) vorgesehen ist, die von der Zuführleitung (9) stromauf des in die Zuführleitung geschalteten Dialysatorventils (16) abzweigt und stromab des Dialysatorventils in die Zuführleitung führt, wobei in die zweite Bypassleitung ein zweites Bypassventil (24) und eine Drossel (25) geschaltet sind.

7. Vorrichtung nach Anspruch 6, **dadurch gekennzeichnet, dass** die Mittel zum Ansteuern der Mittel zum Einstellen des Dialysierflüssigkeitsflusses eine Steuereinheit (18) umfassen, die derart ausgebildet ist, dass in dem Betriebsmodus für den verringerten Dialysierflüssigkeitsfluss das erste und zweite Bypassventil (15,24) geöffnet und das in die Zuführleitung geschaltete Dialysatorventil (16) geschlossen sind.

8. Vorrichtung nach Anspruch 2, **dadurch gekennzeichnet, dass** eine zweite Bypassleitung (26) vorgesehen ist, die von der Abführleitung (12) stromauf des in die Abführleitung geschalteten Dialysatorventils (17) abzweigt und stromab des Dialysatorventils in die Abführleitung führt, wobei in die zweite Bypassleitung ein zweites Bypassventil (27) und eine Drossel (28) geschaltet sind.

9. Vorrichtung nach Anspruch 8, **dadurch gekennzeichnet, dass** die Mittel zum Ansteuern der Mittel zum Einstellen des Dialysierflüssigkeitsflusses eine Steuereinheit (18) umfassen, die derart ausgebildet ist, dass in dem Betriebsmodus für den verringerten Dialysierflüssigkeitsfluss das erste und zweite Bypassventil (15,27) geöffnet und das in die Abführleitung geschaltete Dialysatorventil (17) geschlossen sind.

10. Vorrichtung nach einem der Ansprüche 4 bis 9, **dadurch gekennzeichnet, dass** die Mittel zum Ansteuern der Mittel zum Einstellen des Dialysierflüssigkeitsflusses Mittel (22) zum Messen des Dialysierflüssigkeitsflusses umfassen, wobei die Drossel (21,25,28) einen einstellbaren Strömungsquerschnitt hat.

11. Vorrichtung nach einem der Ansprüche 2 bis 10, **dadurch gekennzeichnet, dass** von der Abführleitung (39) stromauf des in dieselbe geschalteten Dialysatorventils (41) eine erste Probenentnahmeleitung (48) zu dem einen Anschluss (47a) einer Probenentnahmepumpe (47) führt und von dem anderen Anschluss (47b) der Probenentnahmepumpe eine zweite Probenentnahmeleitung (49) zu der Abführleitung (39) stromab des in dieselbe geschalteten Dialysatorventils (41) oder zu der Bypassleitung (42) führt.

12. Vorrichtung nach Anspruch 11, **dadurch gekennzeichnet, dass** von der ersten oder zweiten Probenentnahmeleitung (48, 49) eine dritte Probenentnahmeleitung (52) abgeht, die zu Mitteln (48) zum Bestimmen hämodynamischer Parameter führt.

13. Vorrichtung nach Anspruch 11 oder 12, **dadurch gekennzeichnet, dass** die Probenentnahmenpumpe (47) eine bidirektionale Pumpe ist.

14. Vorrichtung nach Anspruch 11 oder 12, **dadurch gekennzeichnet, dass** die Probeentnahmenpumpe (47) eine unidirektionale Pumpe ist, wobei die erste Probenentnahmeleitung (48) zu dem Sauganschluss und die zweite Probenentnahmeleitung (49) zu dem Druckanschluss führt, und dass von der Zuführleitung (38) oder der Bypassleitung (42) eine vierte Probenentnahmeleitung (60) zu dem Sauganschluss der Probenentnahmepumpe führt.

15. Vorrichtung nach Anspruch 14, **dadurch gekennzeichnet, dass** in die erste und vierte Probenentnahmenleitung (48,60) jeweils ein Probenentnahmenventil (50, 59) geschaltet sind.

16. Vorrichtung nach einem der Ansprüche 11 bis 15, **dadurch gekennzeichnet, dass** die Mittel zum Ansteuern der Mittel zum Einstellen des Dialysierflüssigkeitsflusses eine Steuereinheit (58) umfassen, die derart ausgebildet ist, dass in dem Betriebsmodus für den verringerten Dialysierflüssigkeitsfluss das Bypassventil (43) und das in die Zuführleitung (38) geschaltete Dialysatorventil (40) geöffnet und das in die Abführleitung (40) geschaltete Dialysatorventil (41) geschlossen sind.

## Claims

1. An apparatus for dialysis treatment with
an extracorporeal circuit, into which the blood chamber (3) of a dialyser (1) divided by a semipermeable membrane (2) into the blood chamber and a dialysing fluid chamber (4) is incorporated,
a dialysing fluid supply line (9) of a dialysing fluid path, which supply line is connected to the inlet of the dialysing fluid chamber, and a dialysing fluid discharge line (12), which is connected to the outlet of the dialysing fluid chamber,
a balancing device (10),
a bypass line (14), which branches off from the supply line upstream of the dialysing fluid chamber,
means for the adjustment of the dialysing fluid flow (15, 16, 17; 20, 21; 24, 25; 27, 28), which are provided both in the bypass line and in the supply and/or discharge line, and
means (18) for steering the means for the adjustment of the dialysing fluid flow in the bypass line and the supply line and/or discharge line,
**characterised in that**
the means (18) for steering the means for the adjustment of the dialysing fluid flow (15, 16, 17; 20, 21; 24, 25; 27, 28) are designed in such a way that an operating mode for a reduced dialysing fluid flow through the dialysing fluid chamber (4) can be adjusted, in which mode the means for the adjustment of the dialysing fluid flow both in the bypass line (14) and in the supply and discharge line (9, 12) is steered into a position at least partially releasing the dialysing fluid flow both through the bypass line and through the supply and discharge line, so that a part of the dialysing fluid can be diverted via the bypass line (14).

2. The apparatus according to claim 1, **characterised in that** the means for the adjustment of the dialysing fluid flow includes a bypass valve (15) incorporated into the bypass line and a dialyser valve (16) incorporated into the supply line (9) and/or a dialyser valve (17) incorporated into the discharge line (12).

3. The apparatus according to claim 2, **characterised in that** the means for steering the means for the adjustment of the dialysing fluid flow includes a control unit (18), which is designed in such a way that, in the operating mode for the reduced dialysing fluid flow, the bypass valve (15) and the dialyser valve (16, 17) incorporated into the supply line and into the discharge line respectively are opened.

4. The apparatus according to claim 2, **characterised in that** a second bypass line (19) is provided, which branches off from the first bypass line (14) upstream of the first bypass valve (15) and leads downstream of the first bypass valve to the first bypass line, whereby a second bypass valve (20) and a throttle (21) are incorporated into the second bypass line.

5. The apparatus according to claim 4, **characterised in that** the means for steering the means for the adjustment of the dialysing fluid flow includes a control unit (18), which is designed in such a way that, in the operating mode for the reduced dialysing fluid flow, the first bypass valve (15) is closed and the second bypass valve (20) and the dialyser valve (16, 17) incorporated into the supply line and discharge line respectively are opened.

6. The apparatus according to claim 2, **characterised in that** a second bypass line (23) is provided, which branches off from the supply line (9) upstream of the dialyser valve (16) incorporated into the supply line and leads into the supply line downstream of the dialyser valve, whereby a second bypass valve (24) and a throttle (25) are incorporated into the second bypass line.

7. The apparatus according to claim 6, **characterised in that** the means for steering the means for the adjustment of the dialysing fluid flow includes a control unit (18), which is designed in such a way that, in the operating mode for the reduced dialysing fluid flow, the first and second bypass valves (15, 24) are opened and the dialyser valve (16) incorporated into the supply line is closed.

8. The apparatus according to claim 2, **characterised in that** a second bypass line (26) is provided, which branches off from the discharge line (12) upstream of the dialyser valve (17) incorporated into the discharge line and leads into the discharge line downstream of the dialyser valve, whereby a second bypass valve (27) and a throttle (28) are incorporated into the second bypass line.

9. The apparatus according to claim 8, **characterised in that** the means for steering the means for the adjustment of the dialysing fluid flow includes a control unit (18), which is designed in such a way that, in the operating mode for the reduced dialysing fluid flow, the first and second bypass valves (15, 27) are opened and the dialyser valve (17) incorporated into the discharge line is closed.

10. The apparatus according to any one of claims 4 to 9, **characterised in that** the means for steering the means for the adjustment of the dialysing fluid flow includes means (22) for measuring the dialysing fluid flow, whereby the throttle (21, 25, 28) has an adjustable flow cross-section.

11. The apparatus according to any one of claims 2 to 10, **characterised in that** a first sampling line (48) leads from the discharge line (39) upstream of the dialyser valve (41) incorporated therein to the one connection (47a) of a sampling pump (47) and a second sampling line (49) leads from the other connection (47b) of the sampling pump to the discharge line (39) downstream of the dialyser valve (41) incorporated therein or to the bypass line (42).

12. The apparatus according to claim 11, **characterised in that** there leads away from the first or second sampling line (48, 49) a third sampling line (52), which leads to means (48) for determining haemodynamic parameters.

13. The apparatus according to claim 11 or 12, **characterised in that** the sampling pump (47) is a bi-directional pump.

14. The apparatus according to claim 11 or 12, **characterised in that** the sampling pump (47) is a unidirectional pump, whereby the first sampling line (48) leads to the suction connection and the second sampling line (49) leads to the pressure connection, and **in that** a fourth sampling line (60) leads from the supply line (38) or the bypass line (42) to the suction connection of the sampling pump.

15. The apparatus according to claim 14, **characterised in that** a sampling valve (50, 59) is incorporated into the first and fourth sampling line (48, 60) respectively.

16. The apparatus according to any one of claims 11 to 15, **characterised in that** the means for steering the means for the adjustment of the dialysing fluid flow includes a control unit (58), which is designed in such a way that, in the operating mode for the reduced dialysing fluid flow, the bypass valve (43) and the dialyser valve (40) incorporated into the supply line (38) are opened and the dialyser valve (41) incorporated into the discharge line (40) is closed.

## Revendications

1. Dispositif pour effectuer un traitement de dialyse, comprenant,
un circuit de sang extracorporel dans lequel est montée la chambre de sang (3) d'un dialyseur (1) subdivisé par une membrane semi-perméable (2) en la chambre de sang et une chambre de liquide de dialyse (4),
une conduite d'amenée de liquide de dialyse (9) d'un parcours de liquide de dialyse, qui est reliée à l'entrée de la chambre de liquide de dialyse, et une conduite d'évacuation de liquide de dialyse (12), qui est reliée à la sortie de la chambre de liquide de dialyse,
un système d'établissement de bilan (10),
une conduite de dérivation (14) qui est dérivée de la conduite d'amenée, en amont de la chambre de liquide de dialyse,
des moyens de réglage du flux d'écoulement de liquide de dialyse (15, 16, 17 ; 20, 21 ; 24, 25 ; 27, 28), qui sont prévus aussi bien dans la conduite de dérivation que dans la conduite d'amenée et/ou d'évacuation, et
des moyens (18) pour commander les moyens de réglage du flux d'écoulement de liquide de dialyse dans la conduite de dérivation et la conduite d'amenée et/ou la conduite d'évacuation,
**caractérisé**
**en ce que** les moyens (18) pour commander les moyens de réglage du flux d'écoulement de liquide de dialyse (15, 16, 17 ; 20, 21 ; 24, 25 ; 27, 28) sont réalisés de manière à pouvoir régler un mode de fonctionnement pour un flux d'écoulement de liquide de dialyse réduit à travers la chambre de liquide de dialyse (4), mode dans lequel les moyens de réglage du flux d'écoulement de liquide de dialyse, aussi bien dans la conduite de dérivation (14) que dans la conduite d'amenée et d'évacuation (9, 12), sont commandés pour être dans une position libérant au moins partiellement le flux d'écoulement aussi bien à travers la conduite de dérivation qu'à travers la conduite d'amenée et d'évacuation, de sorte qu'une partie du liquide de dialyse peut être déviée par la conduite de dérivation (14).

2. Dispositif selon la revendication 1, **caractérisé en ce que** les moyens de réglage du flux d'écoulement de liquide de dialyse englobent une vanne de dérivation (15) montée dans la conduite de dérivation, et une vanne de dialyseur (16) montée dans la conduite d'amenée (9) et/ou une vanne de dialyseur (17) montée dans la conduite d'évacuation (12).

3. Dispositif selon la revendication 2, **caractérisé en ce que** les moyens pour commander les moyens de réglage du flux d'écoulement de liquide de dialyse englobent une unité de commande (18), qui est conçue de façon telle, que dans le mode de fonctionnement pour le flux d'écoulement réduit de liquide de dialyse, la vanne de dérivation (15) et la vanne de dialyseur (16, 17) montée dans la conduite d'amenée et respectivement dans la conduite d'évacuation, soient ouvertes.

4. Dispositif selon la revendication 2, **caractérisé en ce qu'**il est prévu une deuxième conduite de dérivation (19), qui est dérivée de la première conduite de dérivation (14) en amont de la première vanne de dérivation (15), et mène à la première conduite de dérivation en aval de la première vanne de dérivation, une deuxième vanne de dérivation (20) et un étranglement (21) étant placés dans la deuxième conduite de dérivation.

5. Dispositif selon la revendication 4, **caractérisé en ce que** les moyens pour commander les moyens de réglage du flux d'écoulement de liquide de dialyse englobent une unité de commande (18) qui est conçue de façon telle, que dans le mode de fonctionnement pour le flux d'écoulement réduit de liquide de dialyse, la première vanne de dérivation (15) soit fermée, et la deuxième vanne de dérivation (20) et la vanne de dialyseur (16, 17) montée dans la conduite d'amenée et respectivement dans la conduite d'évacuation, soient ouvertes.

6. Dispositif selon la revendication 2, **caractérisé en ce que** qu'il est prévu une deuxième conduite de dérivation (23), qui est dérivée de la conduite d'amenée (9) en amont de la vanne de dialyseur (16) montée dans la conduite d'amenée, et mène à la conduite d'amenée en aval de la vanne de dialyseur, une deuxième vanne de dérivation (24) et un étranglement (25) étant montés dans la deuxième conduite de dérivation.

7. Dispositif selon la revendication 6, **caractérisé en ce que** les moyens pour commander les moyens de réglage du flux d'écoulement de liquide de dialyse englobent une unité de commande (18) qui est conçue de façon telle, que dans le mode de fonctionnement pour le flux d'écoulement réduit de liquide de dialyse, la première et la deuxième vanne de dérivation (15, 24) soient ouvertes, et la vanne de dialyseur (16) montée dans la conduite d'amenée soit fermée.

8. Dispositif selon la revendication 2, **caractérisé en ce que** qu'il est prévu une deuxième conduite de dérivation (26), qui est dérivée de la conduite d'évacuation (12) en amont de la vanne de dialyseur (17) montée dans la conduite d'évacuation, et mène à la conduite d'évacuation en aval de la vanne de dialyseur, une deuxième vanne de dérivation (27) et un étranglement (28) étant montés dans la deuxième conduite de dérivation.

9. Dispositif selon la revendication 8, **caractérisé en ce que** les moyens pour commander les moyens de réglage du flux d'écoulement de liquide de dialyse englobent une unité de commande (18) qui est conçue de façon telle, que dans le mode de fonctionnement pour le flux d'écoulement réduit de liquide de dialyse, la première et la deuxième vanne de dérivation (15, 27) soient ouvertes, et la vanne de dialyseur (17) montée dans la conduite d'évacuation soit fermée.

10. Dispositif selon l'une des revendications 4 à 9, **caractérisé en ce que** les moyens pour commander les moyens de réglage du flux d'écoulement de liquide de dialyse englobent des moyens (22) de mesure du flux d'écoulement de liquide de dialyse, l'étranglement (21, 25, 28) présentant une section transversale d'écoulement réglable.

11. Dispositif selon l'une des revendications 2 à 10, **caractérisé en ce que** de la conduite d'évacuation (39), en amont de la vanne de dialyseur (41) qui y est montée, une première conduite de prélèvement d'échantillon (48) mène à l'un des raccords (47a) d'une pompe de prélèvement d'échantillon (47), et de l'autre raccord (47b) de la pompe de prélèvement d'échantillon, une deuxième conduite de prélèvement d'échantillon (49) mène à la conduite d'évacuation (39), en aval de la vanne de dialyseur (41) montée dans celle-ci ou à la conduite de dérivation (42).

12. Dispositif selon la revendication 11, **caractérisé en ce que** de la première ou de la deuxième conduite de prélèvement d'échantillon (48, 49) est issue une troisième conduite de prélèvement d'échantillon (52), qui mène à des moyens (A) pour la détermination de paramètres hémodynamiques.

13. Dispositif selon la revendication 11 ou 12, **caractérisé en ce que** la pompe de prélèvement d'échantillon (47) est une pompe bidirectionnelle.

14. Dispositif selon la revendication 11 ou 12, **caractérisé en ce que** la pompe de prélèvement d'échantillon (47) est une pompe unidirectionnelle, la première conduite de prélèvement d'échantillon (48) menant au raccord d'aspiration et la deuxième conduite de prélèvement d'échantillon (49) au raccord de refoulement, et **en ce qu'**une quatrième conduite de prélèvement d'échantillon (60) mène de la conduite d'amenée (38) ou de la conduite de dérivation (42) au raccord d'aspiration de la pompe de prélèvement d'échantillon.

15. Dispositif selon la revendication 14, **caractérisé en ce que** dans la première et dans la quatrième conduite de prélèvement d'échantillon (48, 60) est montée une vanne respective de prélèvement d'échantillon (50, 59).

16. Dispositif selon l'une des revendications 11 à 15, **caractérisé en ce que** les moyens pour commander les moyens de réglage du flux d'écoulement du liquide de dialyse, englobent une unité de commande (58) qui est conçue de façon telle, que dans le mode de fonctionnement pour le flux d'écoulement réduit de liquide de dialyse, la vanne de dérivation (43) et la vanne de dialyseur (40) montée dans la conduite d'amenée (38) soient ouvertes, et la vanne de dialyseur (41) montée dans la conduite d'évacuation (39) soit fermée.
